(19) Europäisches Patentamt
European Patent Office
Office européen des brevets

(11) **EP 4 343 329 A1**

(12) **EUROPEAN PATENT APPLICATION**

(43) Date of publication:
**27.03.2024 Bulletin 2024/13**

(51) International Patent Classification (IPC):
***G01N 33/58*** (2006.01)

(21) Application number: **23159082.9**

(22) Date of filing: **28.02.2023**

(52) Cooperative Patent Classification (CPC):
**G01N 33/588; G01N 33/582; G01N 33/587**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **26.09.2022 FR 2209748**

(71) Applicant: **Nexdot**
**93230 Romainville (FR)**

(72) Inventors:
• **Gaïtis, Alexandre**
**93230 Romainville (FR)**
• **Rousseau, Loïck**
**93230 Romainville (FR)**
• **Dubertret, Benoît**
**93230 Romainville (FR)**

(74) Representative: **Icosa**
**83 avenue Denfert-Rochereau**
**75014 Paris (FR)**

(54) **NANOPARTICLE STRUCTURES WITH BARCODES**

(57) The present invention relates to a library of barcoded particle population comprising combinations of inorganic fluorescent nanoparticles (10, 11, 12) leading to unique identification of said population.

**FIG. 6**

Processed by Luminess, 75001 PARIS (FR)

**Description**

**FIELD OF INVENTION**

The present invention relates to the field of identification by barcoded structures comprising nanoparticles.

**BACKGROUND OF INVENTION**

Over the past several years, advances in biomedical research technology have driven an unprecedented explosion of biologic data, including genomic and proteomic data, yet the challenge of translating new biomarkers of disease into diagnostics has to be tried. To both validate and deploy the recent discoveries into biological assay requires new approaches to multiplexing and high-throughput biomarker analysis. Few practically available cost-effective assays for multiplexing exist, and new approaches are needed.

Multiplexed assays require that individual probes be reliably identified and tracked throughout an experiment. This identification and tracking are often done using geometrical devices, where the identity of each probe is encoded by its physical position. An alternative approach uses encoded beads, where each probe is attached to a separate bead that is uniquely identifiable.

Bead-based assays offer faster reaction kinetics, increased assay flexibility, and improved reproducibility and reduced costs due to the ability to attach probes to multiple particles at once. However, technical challenges in bead encoding have limited their practical application to date. Existing encoding methods generally fall into two categories: spatial encoding and spectral encoding. Spatial encoding schemes create graphical patterns or bar codes in the particle material in a variety of ways. However, spatial methods face difficulties in cost-effective fabrication, often require large particles to generate large code sets, and have slower and more challenging code readout than existing spectral methods.

Spectral encoding schemes incorporate mixtures of photoluminescent materials that emit light at different wavelengths to generate uniquely identifiable signatures. These schemes allow identification of codes in any orientation and are compatible with conventional bead synthesis procedures and standard detection optics, making them particularly attractive. Despite the promise of spectral encoding schemes, technical challenges have limited their practical code capacity. In particular, designing a library of particles with well separated spectral properties could not reach more than less than a dizain of codes.

The present invention addresses this problem and allow to design libraries with thousands of different codes, opening way to massive multiplexing.

**SUMMARY**

Provided is a library of barcoded particle population in which:

- The barcoded particles are composite particles comprising at least one type of inorganic fluorescent nanoparticle selected from a list of N types of inorganic fluorescent nanoparticles, these inorganic fluorescent nanoparticles being encapsulated in an inorganic material and N being an integer greater than or equal to 3;
- Each population is defined by an N-tuple, wherein the element i of the N-tuple is the weight fraction of the type i of inorganic fluorescent nanoparticle in the composite particles, defining a fluorescence spectrum $I_{(C1,...,Ci,...CN)}(\lambda)$ for that population;
- Each type of the N types of inorganic fluorescent nanoparticles is encapsulated in at least one population; and
- At least one type of inorganic fluorescent nanoparticle is encapsulated in at least two populations with different weight fractions.

In one embodiment, the inorganic fluorescent nanoparticles are distributed throughout the volume of each composite particle.

In one embodiment, the weight fraction of the inorganic fluorescent nanoparticles in the composite particles of at least one population is greater than or equal to 10%.

In one embodiment, the weight fraction of the type i of the inorganic fluorescent nanoparticles in the composite particles is selected from P-tuple $(C_{i1},...C_{iP})$ of predetermined concentrations, wherein P is greater than or equal to 3.

In one embodiment, the inorganic fluorescent nanoparticles are semi-conductive nanoparticles.

In one embodiment, the composite particles are surface modified with biological compounds.

Also provided is an assay kit comprising a library of barcoded particle populations as disclosed hereabove and at least one fluorescent protein.

In one embodiment, the kit comprises composite particles which are surface modified with allergens and the fluorescent protein is able to bind with immunoglobulins.

In one embodiment, the kit comprises composite particles which are surface modified with a biomolecule able to bind with an antibody produced after exposure to a virus and the fluorescent protein is able to bind with immunoglobulins.

In one embodiment, the kit comprises composite particles which are surface modified with a biomolecule able to bind with an envelope glycoprotein of a virus and the at least one fluorescent protein is able to bind with said virus.

In one embodiment, the at least one fluorescent protein is selected from the group of Fluorescein Isothiocyanate (FITC), Peridinin-Chlorophyll Protein Complex (PerCP), Rhodamine, Green Fluorescent Protein (GFP), Yellow Fluorescent Protein (YFP), Propium Iodide, Phycorythrin, Sulforhodamine, Cy5, Cy7, Cy5.5, Cy7.5, DRAQ7.

Also provided is a device for biological assay configured to separate at an individual level the composite particles from an assay kit as disclosed hereabove, dispersed in a biological sample and comprising a reader comprising:

- at least one illumination source; and
- a light detector measuring light intensity and coupled to a spectrometer,
  wherein the at least one illumination source triggers fluorescence of the inorganic fluorescent nanoparticles encapsulated in composite particles and fluorescence of the at least one fluorescent protein.

In one embodiment, the separation at an individual level of the composite particles is operated by introduction of the biological sample in a flow cytometer.

In one embodiment, the separation at an individual level of the composite particles is operated by spreading of the biological sample on a surface.

**DEFINITIONS**

In the present invention, the following terms have the following meanings:

**"Concentration"** refers to the weight fraction of a component in a whole composition. Herein, concentration and weight fraction are equivalent.

**"Core/shell"** refers to heterogeneous nanostructure comprising an inner part: the core, overcoated on its surface, totally or partially, by a film or a layer of at least one atom thick material different from the core: the shell. Core/shell structures are noted as follows: core material/shell material. For instance, a particle comprising a core of CdSe and a shell of ZnS is noted CdSe/ZnS. By extension, core-shell/shell structures are defined as core/first-shell structures overcoated on their surface, totally or partially, by a film or a layer of at least one atom thick material different from the core and/or from the first shell: the second-shell. For instance, a particle comprising a core of CdSe, a first-shell of CdS and a second-shell of ZnS is noted CdSe/CdS/ZnS. Core/shell nanostructure also include nanoparticles in which the central part: the core, is embedded - or encapsulated - by a layer of material disposed on the core: the shell, said shell having a gradient of composition from the core to the outside of the shell. In this case, the composition of the nanoparticle is changing smoothly - for instance continuously - from the core composition - for instance CdSe - to the outside composition of the shell - ZnS for instance. There is no precise boundary between core and shell but properties in centre of the core are different from properties on the outer boundary of shell. In addition, core and shell may have different shapes, for instance a dot - a nanosphere or a nanocube or any other nanocluster - is provided as a core and shell is grown laterally around the core, yielding an heterostructure with shape of a nanoplate but comprising a dot inside the nanoplate.

**"Doped"** refers to a composition of material with a crystalline structure - core or shell - in which a dopant element is substituted to the main element. Such compositions are noted QD:Dopant in the following. For instance, a ZnSe quantum dot may be doped with Manganese (Mn) so that a part of Zn elements are substituted by Mn elements, and noted ZnSe:Mn. The amount of dopant element is preferably less than 10% of the amount of the main element.

**"Encapsulated"** refers to a state in which a material - or a matrix - coats, surrounds, embeds, contains, comprises, wraps, packs, or encloses a plurality of nanoparticles.

**"Fluorescent"** refers to the property of a material that emits light after being excited by absorption of light. Actually, light absorption drives said material in an energetically excited state, which eventually relaxes by emission of light of lower energy, *i.e.,* of longer wavelength - red shifted.

**"FWHM"** refers to Full Width at Half Maximum for a band of emission/absorption of light.

**"Nanometric size"** refers to a size of matter where at least one physical property is directly governed by size. For semi-conductive nanoparticles, nanometric size has to be defined with the average Bohr radius of an electron/hole pair in the material in which quantum effects appear due to confinement. For semi-conductive materials disclosed here, quantum effects appear for size in at least one dimension of the object below 20 nm, preferably below 10 nm, more preferably below 5 nm. For conductive particles, nanometric size has to be defined from the resonance of oscillations of free electron gas density, which becomes relevant for optical measurements in the range from 380 nm to 3 $\mu$m for size in at least one dimension of the object below 20 nm for conductive materials disclosed here.

**"Nanoparticle"** refers to a particle having at least one dimension in the 0.1 to 100 nanometers range. Nanoparticles may have any shape. A nanoparticle may be a single particle or an aggregate of several single particles or a composite particle comprising single particles dispersed in a matrix. Single particles may be crystalline. Single particles may have a core/shell or plate/crown structure.

**"Nanoplatelet"** refers to a nanoparticle having a 2D-shape, i.e. having one dimension smaller than the two others; said smaller dimension ranging from 0.1 to 100 nanometers. In the sense of the present invention, the smallest dimension (hereafter referred to as the thickness) is smaller than the other two dimensions (hereafter referred to as the length and the width) by a factor (aspect ratio) of at least 1.5.

**"Population of particles"** refers to a statistical set of particles having the same properties - maximum emission wavelength, size, composition, structure - inside the normal distribution of particles coming from the manufacturing conditions.

**"Semi-conductive nanoparticles"** refers to particles made of a material having an electronic structure corresponding to semi-conductive materials known in electronic industry but having a **nanometric size.** Due to their specific electronic structure, semi-conductive materials behave as high-pass absorbing materials. Indeed, light having a wavelength more energetic than band gap may be absorbed by the semi-conductive material, yielding an electron/hole pair, an exciton, which later recombine in the material and dissipate heat, or emit light, or both. On the contrary, light having a wavelength less energetic than band gap cannot be absorbed: semi-conductive material is transparent for these wavelengths. In macroscopic semi-conductive materials, visible light is generally absorbed while near/mid infra-red light is not absorbed. When semi-conductive particles have a **nanometric size,** confinement - *i.e.,* shape and nanometric size - governs electronic structure following the rules of quantum mechanics and light absorption may be limited to UV range or UV and high energy visible light. Within this disclosure, **semi-conductive nanoparticles** absorb light having a wavelength below a threshold, this threshold being in the range of 350 nm - 800 nm.

## DETAILED DESCRIPTION

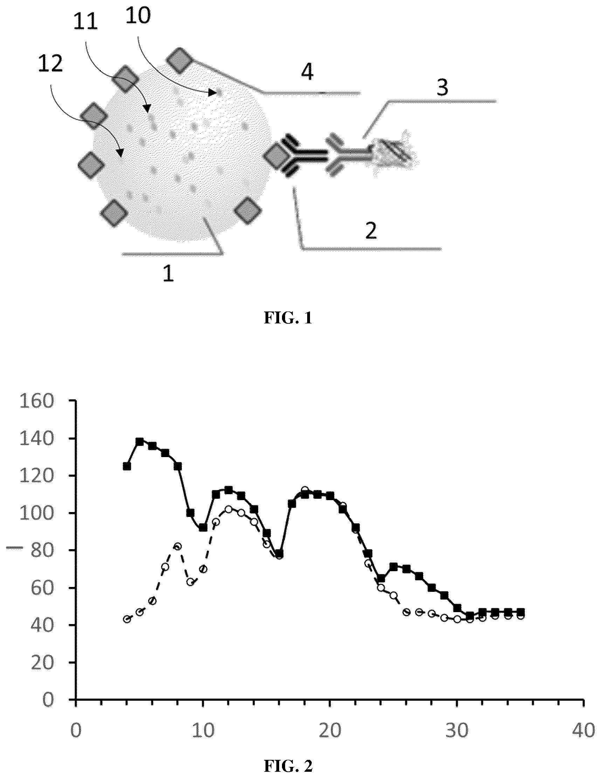

Figure 1 is a diagram representing a composite particle (1) comprising several types of inorganic fluorescent nanoparticles (10, 11, 12) encapsulated in an inorganic matrix. The composite particle (1) is surface functionalized with a particular allergen as an example of a biological compound (4).

When the composite particle (1) is brought into contact with a biological sample, a specific marker, for instance an antibody such as an immunoglobulin (2) present in the sample can specifically bind with the allergen. The specific immunoglobulin (2) may further be associated with a fluorescent protein (3).

Then, the sample can be analyzed, for instance by flow cytometry: when the composite particle (1) is illuminated, the fluorescent light emitted by the protein (3) validates the presence of the selective immunoglobulin (2) associated with an allergen. And the fluorescence associated with the inorganic fluorescent nanoparticles (10, 11, 12) of the composite particle (1) enables identification of the allergen.

In the disclosure, a population of particles is prepared. The particles of this population are composite particles (1) comprising at least one type of inorganic fluorescent nanoparticles (10, 11, 12). The weight fraction of the inorganic fluorescent nanoparticles (10, 11, 12) in the composite particles (1) are the same within a same population. As a result, a single fluorescence spectrum $I_{(C1,...,Ci,...CN)}(\lambda)$ is associated with the population of particles. This spectrum is analogous to a barcode.

Subsequently, a population of particles can be functionalized with a biological compound (4), for example an allergen, such that this particular biological compound (4) will be associated with the unique fluorescence spectrum $I_{(C1,...,Ci,...CN)}(\lambda)$ of the population of particles.

By preparing several populations of particles, characterized by different fluorescence spectra, a library of barcoded particle population is obtained.

Inorganic fluorescent nanoparticles

In this disclosure, the inorganic fluorescent nanoparticles (10, 11, 12) can be any kind of inorganic fluorescent material prepared in the form of nanoparticles.

Suitable inorganic fluorescent nanoparticles (10, 11, 12) may be selected from rare earth-doped nanoparticles. Crystalline matrix of said nanoparticles may be based on vanadate ions ($VO_4^{3-}$). Rare earth dopant may be selected from europium (Eu), dysprosium (Dy), samarium (Sm), praseodymium (Pr), neodymium (Nd), erbium (Er), ytterbium (Yb), cerium (Ce), holmium (Ho), terbium (Tb), thulium (Tm) and mixtures thereof.

Other suitable inorganic fluorescent nanoparticles (10, 11, 12) may be selected from fluorescent semi-conductive nanoparticles known as quantum dots, which may have various composition, shape and structure.

*Quantum dots composition*

In one embodiment, the quantum dots comprise a material of formula

$$M_xQ_yE_zA_w \qquad (I)$$

in which M is selected from the group consisting of Zn, Cd, Hg, Cu, Ag, Au, Ni, Pd, Pt, Co, Fe, Ru, Os, Mn, Tc, Re, Cr, Mo, W, V, Nd, Ta, Ti, Zr, Hf, Be, Mg, Ca, Sr, Ba, Al, Ga, In, Tl, Si, Ge, Sn, Pb, As, Sb, Bi, Sc, Y, La, Ce, Pr, Nd, Sm, Eu, Gd, Tb, Dy, Ho, Er, Tm, Yb, Cs or a mixture thereof; Q is selected from the group consisting of Zn, Cd, Hg, Cu, Ag, Au, Ni, Pd, Pt, Co, Fe, Ru, Os, Mn, Tc, Re, Cr, Mo, W, V, Nd, Ta, Ti, Zr, Hf, Be, Mg, Ca, Sr, Ba, Al, Ga, In, Tl, Si, Ge, Sn, Pb, As, Sb, Bi, Sc, Y, La, Ce, Pr, Nd, Sm, Eu, Gd, Tb, Dy, Ho, Er, Tm, Yb, Cs or a mixture thereof; E is selected from the group consisting of O, S, Se, Te, C, N, P, As, Sb, F, Cl, Br, I, or a mixture thereof; and A is selected from the group consisting of O, S, Se, Te, C, N, P, As, Sb, F, Cl, Br, I, or a mixture thereof. x, y, z and w are independently a decimal number from 0 to 5; x, y, z and w are not simultaneously equal to 0; x and y are not simultaneously equal to 0; z and w are not simultaneously equal to 0.

In particular, quantum dots may comprise a material of formula $M_xE_y$, in which M is Zn, Cd, Hg, Cu, Ag, Al, Ga, In, Si, Ge, Pb, Sb or a mixture thereof; and E is O, S, Se, Te, N, P, As or a mixture thereof. x and y are independently a decimal number from 0 to 5, with the proviso that x and y are not 0 at the same time.

In a specific embodiment, quantum dots comprise a material selected from the group consisting of CdS, CdSe, CdTe, ZnS, ZnSe, ZnTe, HgS, HgSe, HgTe, HgO, GeS, GeSe, GeTe, SnS, SnSe, SnTe, PbS, PbSe, PbTe, $GeS_2$, $GeSe_2$, $SnS_2$, $SnSe_2$, $CuInS_2$, $CuInSe_2$, CuInZnS, CuInZnSe, $AgInS_2$, $AgInSe_2$, CuS, $Cu_2S$, $Ag_2S$, $Ag_2Se$, $Ag_2Te$, FeS, $FeS_2$, InP, $Cd_3P_2$, $Zn_3P_2$, CdO, ZnO, FeO, $Fe_2O_3$, Fe3O4, $Al_2O_3$, $TiO_2$, MgO, MgS, MgSe, MgTe, AlN, AlP, AlAs, AlSb, GaN, GaP, GaAs, GaSb, InN, InP, InAs, InSb, InAsP, TlN, TlP, TlAs, TlSb, $MoS_2$, PdS, Pd4S, $WS_2$, $CsPbCl_3$, $PbBr_3$, $CsPbBr_3$, $CH_3NH_3PbI_3$, $CH_3NH_3PbCl_3$, $CH_3NH_3PbBr_3$, $CsPbI_3$, $FAPbBr_3$, $FAPbI_3$ (where FA stands for formamidinium), or a mixture thereof.

In one embodiment, quantum dots are doped with at least one transition metal such as, for example, Sc, Y, Ti, Zr, Hf, Rf, V, Nb, Ta, Dd, Cr, Mo, W, Sg, Mn, Tc, Re, Bh, Fe, Ru, Os, Hs, Co, Rh, Ir, Ni, Pd, Pt, Cu, Ag or Au. Preferably, quantum dots are doped with Mg, Mn, Al, Cu, Te or Ag. In particular, ZnSe quantum dots doped with Mn :ZnSe:Mn, ZnSe quantum dots doped with Al: ZnSe:Al, or ZnSe quantum dots doped with Mg: ZnSe:Mg are suitable. Amount of dopant is preferably less than 10%. Dopant amount of 5% or 2% are suitable.

*Quantum dots shape*

In one embodiment, quantum dots may have different shapes, provided that they present a nanometric size leading to quantum confinement in the nanoparticle.

Quantum dots may have nanometric sizes in three dimensions, allowing quantum confinement in all three spatial dimensions. Such quantum dots are for instance nanocubes or nanospheres.

Quantum dots may have a nanometric sizes in two dimensions, the third dimension being larger: quantum confinement is in two spatial dimensions. Such quantum dots are for instance nanorods, nanowires or nanorings.

Quantum dots may have a nanometric size in one dimension, the other dimensions being larger: quantum confinement is in one spatial dimension only. Such quantum dots are for instance nanoplatelets, nanosheets, nanoribbons or nanodisks. Nanoplatelets are especially interesting in this disclosure because absorption cross section - *i.e.,* efficiency to capture a photon of incident light on the quantum dot - is ten times higher than a nanosphere having the same composition and structure emitting at the same wavelength. This higher cross section improves significantly sensitivity of assays. In addition, nanoplatelets have narrower FWHM emission spectra - typically below 40 nm - and shorter photoluminescence decay time - by one order of magnitude - as compared to spherical quantum dots

The exact shape of quantum dots defines confinement properties; then electronic and optical properties.

*Quantum dots structure*

In an embodiment, quantum dots are homostructures. By homostructure, it is meant that the quantum dot is homogenous and has the same local composition in all its volume.

In an alternative embodiment, quantum dots are heterostructures. By heterostructure, it is meant that the quantum dot is comprised of several sub-volumes, each sub-volume having a different composition from neighbouring sub-volumes. In a particular embodiment, all sub-volumes have a composition defined by formula (I) disclosed above, with different parameters, *i.e.,* elemental composition and stoichiometry.

Example of heterostructure are core/shell nanoparticles, the core having any shape disclosed above. A shell is a layer covering totally or partially the core. A particular example of core/shell heterostructure is a multi-layered structure comprising a core and several successive shells. For convenience, these multi-layered heterostructures are named core/shell hereafter. Core and shell may have the same shape - sphere in sphere for example - or not - sphere in plate for instance.

Another example of heterostructure are core/crown nanoparticles, the core having any shape disclosed above. A crown is a band of material disposed on the periphery of the core. This heterostructure is particularly useful with cores being nanoplates and crown disposed on the edges of the nanoplate.

These heterostructure may have a gradient of composition from the core to the outside of the shell so that there is no precise boundary between core and shell but properties in centre of the core are different from properties on the outer boundary of shell.

In a configuration, nanoparticles comprise a core based on cadmium, sulfur and selenium and are selected from:

- $CdSe/CdS$, $CdSe/CdS/ZnS$, $CdSe/CdS/ZnSe$, $CdSe/CdS/ZnSe_yS_{(1-y)}$,
- $CdSe_xS_{(1-x)}/ZnS$, $CdSe_xS_{(1-x)}/ZnSe$, $CdSe_xS_{(1-x)}/ZnSe_yS_{(1-y)}$, $CdSe_xTe_{(1-x)}/ZnS$, $CdSe_xTe_{(1-x)}/ZnSe$,
- $CdSe/Cd_yZn_{(1-y)}S$, $CdSe/Cd_yZn_{(1-y)}S/ZnS$, $CdSe/Cd_yZn_{(1-y)}S/ZnSe$, $CdSe/Cd_yZn_{(1-y)}S/ZnSe_zS_{(1-z)}$
- $CdSe/Cd_yZn_{(1-y)}Se$, $CdSe/Cd_yZn_{(1-y)}Se/ZnS$, $CdSe/Cd_yZn_{(1-y)}Se/ZnSe$, $CdSe/Cd_yZn_{(1-y)}Se/ZnSe_zS_{(1-z)}$,
- $CdSe_xS_{(1-x)}/CdS$, $CdSe_xS_{(1-x)}/CdS/ZnS$, $CdSe_xS_{(1-x)}/CdS/ZnSe$, $CdSe_xS_{(1-x)}/CdS/ZnSe_yS_{(1-y)}$,
- $CdSe_xS_{(1-x)}/Cd_yZn_{(1-y)}S$, $CdSe_xS_{(1-x)}/Cd_yZn_{(1-y)}S/ZnS$, $CdSe_xS_{(1-x)}/Cd_yZn_{(1-y)}S/ZnSe$, $CdSe_xS_{(1-x)}/Cd_yZn_{(1-y)}S/ZnSe_zS_{(1-z)}$,
- $CdSe_xS_{(1-x)}/Cd_yZn_{(1-y)}Se$, $CdSe_xS_{(1-x)}/Cd_yZn_{(1-y)}Se/ZnS$, $CdSe_xS_{(1-x)}/Cd_yZn_{(1-y)}Se/ZnSe$, $CdSe_xS_{(1-x)}/Cd_yZn_{(1-y)}Se/ZnSe_zS_{(1-z)}$,

where x, y and z are rational numbers between 0 (excluded) and 1 (excluded), and emit light by fluorescence. Emitted light is typically a band centered on a wavelength in the visible range from 380 nm to 780 nm. Emitted light is typically a band having a FWHM less than 50 nm, preferably less than 30 nm, more preferably less than 20 nm.

In a configuration, nanoparticles comprise a core based on zinc, sulfur and selenium and are selected from:

- $ZnSe/ZnS$, $ZnSe/ZnSe_yS_{(1-y)}$, $ZnTe/ZnSe_yS_{(1-y)}$
- $ZnSe_xS_{(1-x)}/ZnS$, $ZnSe_xS_{(1-x)}/ZnSe$, $ZnSe_xS_{(1-x)}/ZnSe_yS_{(1-y)}$, $ZnSe_xTe_{(1-x)}/ZnS$, $ZnSe_xTe_{(1-x)}/ZnSe$, $ZnSe_xTe_{(1-x)}/ZnSe_xS_{(1-x)}$,
- $ZnSe/Cd_yZn_{(1-y)}S$, $ZnSe/Cd_yZn_{(1-y)}S/ZnS$, $ZnSe/Cd_yZn_{(1-y)}S/ZnSe$, $ZnSe/Cd_yZn_{(1-y)}S/ZnSe_zS_{(1-z)}$
- $ZnSe/Cd_yZn_{(1-y)}Se$, $ZnSe/Cd_yZn_{(1-y)}Se/ZnS$, $ZnSe/Cd_yZn_{(1-y)}Se/ZnSe$, $ZnSe/Cd_yZn_{(1-y)}Se/ZnSe_zS_{(1-z)}$,
- $ZnSe_xS_{(1-x)}/ZnS$, $ZnSe_xS_{(1-x)}/ZnS/ZnSe$, $ZnSe_xS_{(1-x)}/ZnS/ZnSe_yS_{(1-y)}$,
- $ZnSe_xS_{(1-x)}/Cd_yZn_{(1-y)}S$, $ZnSe_xS_{(1-x)}/Cd_yZn_{(1-y)}S/ZnS$, $ZnSe_xS_{(1-x)}/Cd_yZn_{(1-y)}S/ZnSe$, $ZnSe_xS_{(1-x)}/Cd_yZn_{(1-y)}S/ZnSe_zS_{(1-z)}$,
- $ZnSe_xS_{(1-x)}/Cd_yZn_{(1-y)}Se$, $ZnSe_xS_{(1-x)}/Cd_yZn_{(1-y)}Se/ZnS$, $ZnSe_xS_{(1-x)}/Cd_yZn_{(1-y)}Se/ZnSe$, $ZnSe_xS_{(1-x)}/Cd_yZn_{(1-y)}Se/ZnSe_zS_{(1-z)}$,

where x, y and z are rational numbers between 0 (excluded) and 1 (excluded), and emit light by fluorescence. Emitted light is typically a band centered on a wavelength in the visible range from 380 nm to 780 nm. Emitted light is typically a band having a FWHM less than 50 nm, preferably less than 30 nm, more preferably less than 20 nm.

In a configuration, nanoparticles comprise a core based on zinc, cadmium, sulfur and selenium and are selected from:

- $Cd_wZn_{(1-w)}Se/CdS$, $Cd_wZn_{(1-w)}Se/CdS/ZnS$, $Cd_wZn_{(1-w)}Se/CdS/ZnSe$, $Cd_wZn_{(1-w)}Se/CdS/ZnSe_yS_{(1-y)}$,
- $Cd_wZn_{(1-w)}Se_xS_{(1-x)}/ZnS$, $Cd_wZn_{(1-w)}Se_xS_{(1-x)}/ZnSe$, $Cd_wZn_{(1-w)}Se_xS_{(1-x)}/ZnSe_yS_{(1-y)}$, $Cd_wZn_{(1-w)}Se_xTe_{(1-x)}/ZnS$, $Cd_wZn_{(1-w)}Se_xTe_{(1-x)}/ZnSe$,
- $Cd_wZn_{(1-w)}Se/Cd_yZn_{(1-y)}S$, $Cd_wZn_{(1-w)}Se/Cd_yZn_{(1-y)}S/ZnS$, $Cd_wZn_{(1-w)}Se/Cd_yZn_{(1-y)}S/ZnSe$, $Cd_wZn_{(1-w)}Se/Cd_yZn_{(1-y)}S/ZnSe_zS_{(1-z)}$
- $Cd_wZn_{(1-w)}Se/Cd_yZn_{(1-y)}Se$, $Cd_wZn_{(1-w)}Se/Cd_yZn_{(1-y)}Se/ZnS$, $Cd_wZn_{(1-w)}Se/Cd_yZn_{(1-y)}Se/ZnSe$, $Cd_wZn_{(1-w)}Se/Cd_yZn_{(1-y)}Se/ZnSe_zS_{(1-z)}$,
- $Cd_wZn_{(1-w)}Se_xS_{(1-x)}/CdS$, $Cd_wZn_{(1-w)}Se_xS_{(1-x)}/CdS/ZnS$, $Cd_wZn_{(1-w)}Se_xS_{(1-x)}/CdS/ZnSe$, $Cd_wZn_{(1-w)}Se_xS_{(1-x)}/CdS/ZnSe_yS_{(1-y)}$,
- $Cd_wZn_{(1-w)}Se_xS_{(1-x)}/Cd_yZn_{(1-y)}S$, $Cd_wZn_{(1-w)}Se_xS_{(1-x)}/Cd_yZn_{(1-y)}S/ZnS$, $Cd_wZn_{(1-w)}Se_xS_{(1-x)}/Cd_yZn_{(1-y)}S/ZnSe$, $Cd_wZn_{(1-w)}Se_xS_{(1-x)}/Cd_yZn_{(1-y)}S/ZnSe_zS_{(1-z)}$,
- $Cd_wZn_{(1-w)}Se_xS_{(1-x)}/Cd_yZn_{(1-y)}Se$, $Cd_wZn_{(1-w)}Se_xS_{(1-x)}/Cd_yZn_{(1-y)}Se/ZnS$, $Cd_wZn_{(1-w)}Se_xS_{(1-x)}/Cd_yZn_{(1-y)}Se/ZnSe$, $Cd_wZn_{(1-w)}Se_xS_{(1-x)}/Cd_yZn_{(1-y)}Se/ZnSe_zS_{(1-z)}$,

where w, x, y and z are rational numbers between 0 (excluded) and 1 (excluded), and emit light by fluorescence. Emitted light is typically a band centered on a wavelength in the visible range from 380 nm to 780 nm. Emitted light is typically a band having a FWHM less than 50 nm, preferably less than 30 nm, more preferably less than 20 nm.

Other particularly suitable nanoparticles are selected from InP/ZnS, InP/ZnSe, InP/ZnSe$_x$S$_{(1-x)}$, InP/GaP, Cu$_x$In$_y$Zn$_{(1-x-y)}$S/ZnS, In$_x$As$_{(1-x)}$P/ZnSe$_x$S$_{(1-x)}$, CsPbBr$_3$, CsPbI$_3$, FAPbI$_3$, where FA stands for formamidinium and x and y are rational numbers between 0 (excluded) and 1 (excluded).

Other particularly suitable nanoparticles are selected from doped quantum dots as core, such as ZnSe:Mn/ZnS, or ZnSe:Cu/ZnS.

Other particularly suitable nanoplatelets are selected from ZnTe/ZnSe$_y$S$_{(1-y)}$, ZnSe$_x$Te$_{(1-x)}$/ZnS, ZnSe$_x$Te$_{(1-x)}$/ZnSe, ZnSe$_x$Te$_{(1-x)}$/ZnSe$_y$S$_{(1-y)}$, where x and y are rational numbers between 0 (excluded) and 1 (excluded).

Suitable nanoparticles emitting deep-red light at 700nm with FWHM below 25 nm are cadmium bases nanoplatelets core/shell/shell nanoplatelet CdSe/CdS/ZnS, with a core of thickness of 1.2 nm and a lateral dimension, *i.e.,* length or width, greater than 8 nm and thicknesses of shells are respectively 3.5 nm and 0.5nm.

Suitable nanoparticles emitting red light at 653 nm with FWHM of 25 nm are core/shell/shell nanoplatelets of CdSe$_{0.72}$S$_{0.28}$/CdS/ZnS, with a core of thickness 1.2 nm and a lateral dimension, *i.e.,* length or width, greater than 8 nm and shells of thicknesses 3.5 nm and 0.5 nm.

Suitable nanoparticles emitting red light at 630 nm with FWHM of 25 nm are core/shell/shell nanoplatelets of CdSe$_{0.45}$S$_{0.55}$/Cd$_{0.30}$Zn$_{0.70}$S/ZnS, with a core of thickness 1.2 nm and a lateral dimension, *i.e.,* length or width, greater than 8 nm and shells of thicknesses 2.5 nm and 2 nm.

Suitable nanoparticles emitting red light at 630 nm with FWHM of 40 nm are core/shell nanospheres of InP/ZnS, with a core of diameter 3.5 nm and shell thicknesses of 2 nm.

Suitable nanoparticles emitting red light at 607 nm with FWHM of 23 nm are nanoplatelets core/shell of CdSe/ZnS with a core thickness of 1,2 nm and a lateral dimension, *i.e.,* length or width, greater than 10 nm. The shell thickness is 1,25 nm.

Suitable nanoparticles emitting orange light at 580 nm with FWHM of 21 nm are nanoplatelets core/shell of CdSe/ZnS with a core thickness of 1,2 nm and a lateral dimension, *i.e.,* length or width, greater than 10 nm. The shell thickness is 1 nm.

Suitable nanoparticles emitting green light at 565 nm are core/shell/shell nanoplatelets of CdSe$_{0.10}$S$_{0.90}$/Cd$_{0.20}$Zn$_{0.80}$S, with a core of thickness 1.5 nm and a lateral dimension, *i.e.,* length or width, greater than 10 nm and shells of thickness 2.5 nm.

Suitable nanoparticles emitting orange light at 545 nm with FWHM of 70 nm are nanospheres of ZnSe doped with Mn noted ZnSe:Mn, having a diameter of 4.3 nm.

Suitable nanoparticles emitting green light at 530 nm are core/shell/shell nanoplatelets of CdSe$_{0.10}$S$_{0.90}$/ZnS/Cd$_{0.20}$Zn$_{0.80}$S, with a core of thickness 1.5 nm and a lateral dimension, *i.e.,* length or width, greater than 10 nm and shells of thicknesses 1 nm and 2.5 nm.

Suitable nanoparticles emitting green light at 515 nm with FWHM below 25 nm are core/shell nanospheres of CdSe/ZnS, core CdSe has a 4.5 nm radius and ZnS shell has a 2 nm thickness.

Suitable nanoparticles emitting green light at 505 nm with FWHM of 50 nm are core/shell nanospheres of InP/ZnSeS, having a diameter of 4.5 nm

Suitable nanoparticles emitting sky-blue light at 490 nm with FWHM of 25 nm are core/shell/shell nanospheres of Cd$_{0.2}$Zn$_{0.8}$Se/Cd$_{0.3}$Zn$_{0.7}$Se$_{0.4}$S$_{0.6}$/ZnS. The core has a radius of 2,4 nm whereas the first shell has a thickness of 0.5 nm and the last shell, 0.7 nm.

Suitable nanoparticles emitting sky-blue light at 480 nm with FWHM of 25 nm are core/shell/shell nanospheres of Cd$_{0.2}$Zn$_{0.8}$Se/Cd$_{0.3}$Zn$_{0.7}$Se$_{0.4}$S$_{0.6}$/ZnS. The core has a radius of 2,1 nm whereas the first shell has a thickness of 0.5 nm and the last shell, 0.7 nm.

Suitable nanoparticles emitting sky-blue light at 470 nm with FWHM of 25 nm are core/shell/shell nanospheres of Cd$_{0.2}$Zn$_{0.8}$Se/Cd$_{0.3}$Zn$_{0.7}$Se$_{0.4}$S$_{0.6}$/ZnS. The core has a radius of 1,8 nm whereas the first shell has a thickness of 0.5 nm and the last shell, 0.7 nm.

Suitable nanoparticles emitting blue light at 455 nm with FWHM of 30 nm are core/shell/shell nanospheres of ZnSe$_{0.94}$Te$_{0.06}$/ZnSe/ZnS with a core of 3.1 nm diameter. The first shell of ZnSe has a thickness of 2.6 nm and the second shell of ZnS has a thickness of 1.25 nm. The total size of those core/shell/shell nanospheres is about 11 nm.

Suitable nanoparticles emitting blue light at 450 nm with FWHM of 30 nm are core/shell nanoplatelets of CdS/ZnS, with a core of thickness 0.9 nm and a lateral dimension, *i.e.,* length or width, greater than 15 nm and a shell of thickness 1 nm.

Suitable nanoparticles emitting blue light at 450 nm with FWHM of 30 nm are core/shell nanospheres of Cd$_{0.5}$Zn$_{0.5}$S/ZnS, with a core of 6.3 nm diameter and a shell thickness of 2.6 nm. The emission may be adapted from 435 to 460 nm for these core/shell nanospheres by varying the ratio of Cd/Zn precursors of the core.

Suitable nanoparticles emitting blue light at 445 nm with FWHM of 10 nm are core/shell nanospheres ZnSe/ZnS with a core of 4 nm radius and a shell thickness of 2 nm.

Suitable nanoparticles emitting blue light at 430 nm with FWHM of 20 nm are core/shell nanospheres of ZnSe/ZnS, having a core diameter of 4.5 nm and a 2 nm shell thickness.

Suitable nanoparticles emitting violet light at 405 nm with FWHM of 15 nm are core/shell nanospheres of ZnSe/ZnS, with a core of 2,7 nm diameter and a shell thickness of 2 nm.

Suitable nanoparticles emitting violet light at 395 nm with FWHM of 15 nm are core/shell nanospheres of $ZnSe_{0.9}S_{0.1}/ZnS$, with a core of 2,4 nm diameter and a shell thickness of 2 nm.

Composite particles - Encapsulated quantum dots

In this disclosure, inorganic fluorescent nanoparticles (10, 11, 12) are encapsulated in a matrix, preferably an inorganic matrix, more preferably an oxide matrix, so as to form composite particles (1).

In an embodiment, the composite particle (1) has a mean size greater than 50 nm and less than 1000 nm, preferably greater than 150 nm. The shape of the composite particle (1) may be spheric, or anisotropic.

The matrix of the composite particles may be inorganic and selected in the group of $SiO_2$, $Al_2O_3$, $TiO_2$, $ZrO_2$, ZnO, $HfO_2$, MgO, $SnO_2$, $Nb_2O_5$, or $CeO_2$. They can be prepared from precursors in a process known as Sol-Gel. The method of preparation disclosed in international patent application PCT/EP2018/064439 is especially suitable.

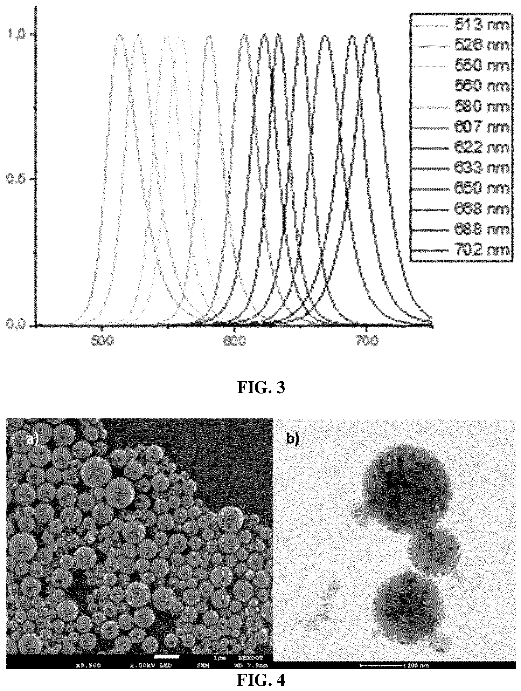

Composite particles (1) comprise at least one type of inorganic fluorescent nanoparticles (10, 11, 12). In order to obtain a library with a larger number of different populations of barcoded particles, inorganic fluorescent nanoparticles (10, 11, 12) are selected from a list of N types of inorganic fluorescent nanoparticles (10, 11, 12), with N an integer greater than or equal to 3. N may be 4, 5, 6, 7, 8, 9, 10, 11 or 12. Figure 3 shows the fluorescence spectra of 12 different inorganic fluorescent nanoparticles (10, 11, 12) with fluorescence peaks well separated in the visible range.

In an embodiment, the weight fraction of inorganic fluorescent nanoparticles (10, 11, 12) in composite particle (1) is greater than 0.5%. In a preferred embodiment, weight fraction of inorganic fluorescent nanoparticles (10, 11, 12) in composite particle (1) is greater than 5%, more preferably greater than 10%, ideally greater than 15%. By weight fraction of inorganic fluorescent nanoparticles (10, 11, 12), it is meant the weight fraction of all inorganic fluorescent nanoparticles (10, 11, 12), *i.e.,* of several different types usually.

Indeed, light emitted by composite particle (1) is the sum of optical conversion of inorganic fluorescent nanoparticles (10, 11, 12) comprised in said composite particle: a high weight fraction corresponds to higher absorption of light and higher fluorescence. However, in this disclosure, the objective is not only to obtain a high fluorescence but to obtain a library of barcoded particles whose fluorescence spectra are well separated. The separation is:

- based on spectral properties of fluorescence: fluorescence peaks and FWHM for each type of inorganic fluorescent nanoparticles (10, 11, 12); and
- based on intensity of fluorescence: the concentration of each type of inorganic fluorescent nanoparticles (10, 11, 12) defines an intensity of fluorescence.

*Spectral separation*

The sensitivity of spectrophotometer enables to separate very precisely light wavelengths down to one nanometer. When such a precision is not required, the spectrophotometer may acquire signal by channels: each channel correspond to a predetermined range of wavelengths in which incident light is summed. The relevant part of visible spectrum may thus be covered with a limited number of channels, typically between 20 and 50. Channels may be adjacent, *i.e.,* with adjacent ranges of wavelength, or not, *i.e.,* discrete. Besides, inorganic fluorescent nanoparticles (10, 11, 12) emit light within an emission spectrum which is not a Dirac impulsion, but a spectrum defined by a peak - maximum of light emission - and a FWHM. Emission peak and width of the emission spectrum is governed by intrinsic properties of inorganic fluorescent nanoparticles (10, 11, 12): composition, structure, crystallinity... Practically, emission spectra from two types of inorganic fluorescent nanoparticles (10, 11, 12) are well separated spectrally if their emission peaks are separated by a distance greater than the half of the sum of their FWHM. In these conditions, the emission peak of one type of inorganic fluorescent nanoparticles (10, 11, 12) is outside the main range of wavelength of the emission spectrum of the other type of inorganic fluorescent nanoparticles (10, 11, 12). This spectral separation condition may be influenced by the number of channels of the spectrophotometer. Preferably, the spectrophotometer is selected with channels having ranges of wavelength less than the FWHM of emission spectra.

It is therefore preferable to use inorganic fluorescent nanoparticles (10, 11, 12) with emission spectrum having a low FWHM: this feature is obtained with inorganic fluorescent nanoparticles (10, 11, 12) having a nanoplatelet shape, which is preferred in this disclosure.

*Intensity separation*

Two composite particles (1) comprising the same type of inorganic fluorescent nanoparticles (10, 11, 12) will have similar emission spectrum - peak and FWHM - but may differ in intensity. Obviously, a composite particle (1) with a weight fraction of inorganic fluorescent nanoparticles (10, 11, 12) of $C$ will emit half the light of the same - in size, structure and composition - composite particle (1) with a weight fraction of inorganic fluorescent nanoparticles (10, 11, 12) of $2 \times C$.

However, in a population of composite particles (1), the weight fraction of inorganic fluorescent nanoparticles (10, 11, 12) is not exactly constant but distributed statistically - because composite particles (1) are not strictly a monodisperse population for instance. Assuming that preparation of composite particles (1) leads to a gaussian distribution of weight fraction of inorganic fluorescent nanoparticles (10, 11, 12), the mean weight fraction will correspond to a number $N_0/2$ of inorganic fluorescent nanoparticles (10, 11, 12) in one composite particle, and standard deviation will be $\sigma =$

$\sqrt{N_0/2}$. Intensity of fluorescence light will follow the same distribution.

Two populations of composite particles (1) will be separated in intensity if distance between the mean intensities of the two population is greater than half of the sum of the standard deviations of each population. In other words, if a first population of composite particles (1) has a mean weight fraction corresponding to a number $N_n/2$ of inorganic fluorescent nanoparticles (10, 11, 12) and a second population of composite particles (1) has a mean weight fraction corresponding to a number $N_{n+1}/2$ of inorganic fluorescent nanoparticles (10, 11, 12), intensity of emitted light will be well separated if $N_n/2 + \sqrt{N_n/2} < N_{n+1}/2 - \sqrt{N_{n+1}/2}$. This condition provides with the difference in weight fraction of inorganic fluorescent nanoparticles (10, 11, 12) for two populations of composite particles (1) leading to separation in intensity: $N_{n+1} > N_n + 2\sqrt{2N_n} + 2$.

For better separation, one may define a stricter condition, using a multiple of standard deviation $\sigma$.

With $2\sigma$ condition, two populations of composite particles (1) leading to separation in intensity shall respect:

$$N_{n+1} > N_n + 4\sqrt{2N_n} + 8.$$

With $3\sigma$ condition, two populations of composite particles (1) leading to separation in intensity shall respect:

$$N_{n+1} > N_n + 6\sqrt{2N_n} + 18.$$

Table I below shows the weight fraction scales, *i.e.*, the proportion in which weight fractions have to be defined, leading to good separation of successive population of composite particles (1), for $\sigma$, $2\sigma$ and $3\sigma$ conditions:

Table I: Scales of weight fraction of inorganic fluorescent nanoparticles (10, 11, 12) leading to good separation in intensity.

|  | $N_0$ | $N_1$ | $N_2$ | $N_3$ | $N_4$ | $N_5$ | $N_6$ | $N_7$ | $N_8$ | $N_9$ |
|---|---|---|---|---|---|---|---|---|---|---|
| $\sigma$ | 100 | 131 | 166 | 205 | 248 | 295 | 346 | 401 | 460 | 523 |
| $2\sigma$ | 100 | 165 | 246 | 343 | 456 | 585 | 730 | 891 | 1068 | 1261 |
| $3\sigma$ | 100 | 203 | 342 | 517 | 728 | 975 | 1258 | 1577 | 1932 | 2323 |

From Table I, it appears clearly that ten well separated populations of composite particles (1) can be obtained, within a $\sigma$ condition, with relative weight fractions of inorganic fluorescent nanoparticles (10, 11, 12) distributed between 1 and 5. In other words, populations of composite particles (1) - with the same size, structure and composition - comprising 10%, 13%, 16.5%, 20.5%, 25%, 29.5%, 35%, 40%, 46% and 52% in weight of the same type of inorganic fluorescent nanoparticles (10, 11, 12) will be well separated. Composite particles with concentrations divided by ten, but in the same proportions, will be also well separated.

Similarly, five populations may be well separated within a $2\sigma$ condition with relative weight fractions of inorganic fluorescent nanoparticles (10, 11, 12) distributed between 1 and 5. And four populations may be well separated within a very strict $3\sigma$ condition with relative weight fractions of inorganic fluorescent nanoparticles (10, 11, 12) distributed between 1 and 5.

These scales define the relative values of the predetermined concentrations in which the weight fraction of inorganic fluorescent nanoparticles (10, 11, 12) may be selected to have a good separation in intensity. Scales with greater differences between successive weight fractions are suitable. These scales are noted in the form of a P-tuple $(C_{i1},...C_{iP})$ of predetermined weight fractions for the inorganic fluorescent nanoparticles (10, 11, 12) of type i. Typically the predetermined concentrations may be in the range of 0.1% - 30%. Preferably, the predetermined concentrations may be in the range of 0.1% - 10%, so that the maximum predetermined concentrations of each type of inorganic fluorescent nanoparticles (10, 11, 12) can be used in composite particles (1) leading to a concentration of all inorganic fluorescent nanoparticles (10, 11, 12) in composite particles (1) below 40% for four different type of inorganic fluorescent nanoparticles (10, 11, 12), or below 50% for five different type of inorganic fluorescent nanoparticles (10, 11, 12).

Populations of composite particles and libraries

In this disclosure, several populations of composite particles (1) are gathered to form a library. First, a set of N types of inorganic fluorescent nanoparticles (10, 11, 12) is selected. Preferably, these N types of inorganic fluorescent nanoparticles (10, 11, 12) are spectrally separated. Second, each population is defined by a composition - in type and in weight fraction - of inorganic fluorescent nanoparticles (10, 11, 12), noted as a N-tuple (*C1*, ..., *Ci*, ... *CN*) where *Ci* denotes the weight fraction of inorganic fluorescent nanoparticles (10, 11, 12) of type i in the composite particle. Said composition defines a fluorescence spectrum $I_{(C1,...,Ci,...CN)}(\lambda)$, with wavelength of emission peaks associated to each type of inorganic fluorescent nanoparticles (10, 11, 12) and intensities of emission peaks associated with the weight fraction of said inorganic fluorescent nanoparticles (10, 11, 12). In a specific embodiment, *C*1 may be null, *i.e.,* one inorganic fluorescent nanoparticle is absent from the composite particle. Such a condition increases the possible combinations without increasing the weight fraction of inorganic fluorescent nanoparticles (10, 11, 12) in composite particles (1). Of course, an "empty" composite particle, without any inorganic fluorescent nanoparticle will not be used.

In order to have a library enabling sufficient variations, each type of the N types of inorganic fluorescent nanoparticles (10, 11, 12) is encapsulated in at least one population and at least one type of inorganic fluorescent nanoparticle is encapsulated in at least two populations with different concentrations.

The weight fraction of inorganic fluorescent nanoparticles (10, 11, 12) in composite particles (1) of a given population is then the sum of the weight fraction of each type of inorganic fluorescent nanoparticles (10, 11, 12). Composite particles (1) comprising several types of inorganic fluorescent nanoparticles (10, 11, 12) with high weight fractions may have a total weight fraction of inorganic fluorescent nanoparticles (10, 11, 12) greater than 10%, or greater than 20%, or greater than 30%, even greater than 40%.

In an embodiment, weight fractions of inorganic fluorescent nanoparticles (10, 11, 12) are selected in a scale of weight fractions as defined hereabove as P-tuple $(C_{i1},...C_{iP})$ in order to improve separation in intensity. In this embodiment, P is greater than or equal to 3, preferably greater than or equal to 5. When $\sigma$ condition for separation in intensity is used, P may be greater or equal to 8.

Finally, the design of a library results from a compromise between the number of populations desired which may reach $P^N$ with N the number of types of inorganic fluorescent nanoparticles (10, 11, 12) and P the number of weight fractions available for each type of inorganic fluorescent nanoparticles (10, 11, 12). For instance, 5 types of inorganic fluorescent nanoparticles (10, 11, 12) with 4 weight fractions leads to a library of 1024 different populations which can be separated appropriately: this is the configuration used in example 2 below.

For the design of a library, three main criteria have to be considered. First, the number of different populations of the library leads to the selection of N - number of inorganic fluorescent nanoparticles (10, 11, 12) used - and P - number of different concentrations of inorganic fluorescent nanoparticles (10, 11, 12). However, the N types of inorganic fluorescent nanoparticles (10, 11, 12) need to be well separated - spectrally and in intensity - leading to a limit for N. In addition, with a given N and P, a population shall be comprising high concentrations of all inorganic fluorescent nanoparticles (10, 11, 12) - $C_{iP}$ for all i values from 1 to N - leading to a very high weight fraction of inorganic fluorescent nanoparticles (10, 11, 12). However, this weight fraction cannot exceed 60%, preferably 50%, in order to have a good dispersion of inorganic fluorescent nanoparticles (10, 11, 12) in the composite particles (1). And the choice on separation in intensity has an impact on the scale of weight fractions. Finally, there is a tradeoff between these criteria to obtain a suitable library.

Last, it is required that during assay, all populations are present in the sample under assay, with a sufficient number of composite particles (1) to enable detection.

In an embodiment, a library may not contain all the $P^N$ possible combinations enabled by N inorganic fluorescent nanoparticles (10, 11, 12) and scales of weight fraction with P values. The heavy charged populations having high weight fraction for some or all types of inorganic fluorescent nanoparticles (10, 11, 12) may be omitted. Or, scales of weight fraction may be of different length for various scales of weight fraction. Or, scales of weight fraction may correspond to different conditions of separation in intensity with $3\sigma$, $2\sigma$ or $\sigma$ condition.

Libraries with N comprised between 3 and 6 and P comprised between 3 and 5 are especially adapted to biological assay. The smallest library comprises $3^3 = 9$ populations, whereas the largest library comprises $5^6 = 15625$

populations.

Assay kit

Another object of this disclosure is an assay kit comprising a library of barcoded particle populations and at least one fluorescent protein (3).

Such an assay kit is specifically designed to check if a biological sample contains a compound of interest.

First, barcoded particle populations are selected in a library. Composite particles of each population are further surface modified with a specific biological compound (4) - or biomolecule, which may be an allergen, a nucleic acid, a protein or a recombinant protein for instance - some of them being able to interact with the compound of interest.

Second, at least one fluorescent protein (3) able to associate with the compound of interest is selected. The number of fluorescent proteins (3) depends on the number of compounds of interest targeted in the sample and depends on the ability of the fluorescent proteins (3) to associate with different compounds of interest. Suitable fluorescent proteins (3) may comprise a fluorescent moiety linked to an antibody or a nanobody such as $V_HH$ fragments, the latter being able to bind the compound of interest. Suitable fluorescent proteins (3) or fluorescent moieties may be purchased from Fluorofinder (Bloomfield), by proper selection of fluorescent dye in the catalog. Particularly suitable fluorescent proteins are Fluorescein Isothiocyanate (FITC), Peridinin-Chlorophyll Protein Complex (PerCP), Rhodamine, Green Fluorescent Protein (GFP), Yellow Fluorescent Protein (YFP), Propium Iodide, Phycorythrin, Sulforhodamine, Cy5, Cy7, Cy5.5, Cy7.5, DRAQ7 or any derivative thereof.

Then, the biological sample is mixed with the barcoded particles and the fluorescent protein (3), leading eventually to a complex - illustrated in figure 1 - comprising a composite particle (1) bound to the compound of interest via the biological compound (4) grafted on its surface and the fluorescent protein (3) associated to the compound of interest. This complex is then easily detected and identified by a reader as disclosed hereafter.

In an embodiment, the assay kit is designed to identify allergies of a patient. Each population of barcoded particles is surface modified with an allergen. This allergen is able to be bound by an immunoglobulin (2) present in the biological sample. Last, the immunoglobulin (2) is bound by a fluorescent protein (3), leading to the complex of figure 1. Examples of allergen are given in example 2.

In an embodiment, the assay kit is designed for serology and enables the detection of the viral infectious past exposure of a patient. Each population of barcoded particles is surface modified with a biomolecule (4) able to bind with an antibody produced after exposure to a virus. This biomolecule (4) is able to be bound by an antibody present in the biological sample. Last, the antibody is bound by a fluorescent protein (3), leading to the complex of figure 1. Typically, the antibody may be an immunoglobulin (2).

In an embodiment, the assay kit is designed for serology and enables the detection of the viral infectious status of a patient. Each population of barcoded particles is surface modified with a biomolecule (4) able to bind with an envelope glycoprotein of a virus. This biomolecule (4) is able to be bound by an active virus present in the biological sample. Last, a fluorescent protein (3) may be modified with a nucleic acid sequence to bind with another site of the virus, leading to the complex of figure 1. Examples of viral envelope glycoproteins - envelope proteins (E), membrane proteins (M) or spike proteins (S) - are the Vesicular stomatitis virus VSV-G glycoprotein, the Murine leukemia virus (MLV) envelope glycoprotein, the Ross river virus (RRV) E2 glycoprotein, the baculovirus gp64 glycoprotein, the Influenza virus Hemagglutinin (HA) and Neuraminidase (NA), the SARS-CoV Spike (S) glycoprotein, the Hepatitis C virus E1 and E2 glycoproteins, the Human immunodeficiency virus gp120, gp41 and gp160 glycoproteins, the Ebola virus GP1 and GP2 Spike glycoproteins, the Dengue virus E protein, the Chikungunya virus E1 and E2 proteins, or the Herpes simplex virus gB glycoprotein.

Similarly, the biomolecules (4) may be able to bind with another structure of a virus - capside, deactivated virus, envelope, nucleic material under single strand or double strand form...

In an embodiment, the assay kit is designed for serology and enables the detection of the bacterial infectious status of a patient. Each population of barcoded particles is surface modified with a biomolecule (4) able to bind with a metabolite of a specific bacteria. Last, a modified fluorescent protein (3) able to bind with the metabolite is used, leading to the complex of figure 1

Readers - Device for biological assay

Another object of this disclosure is a reader enabling sample analysis. The reader comprises at least one illumination source and a light detector measuring light intensity coupled to a spectrometer. During analysis, the sample in which the populations of composite particles (1) grafted with biological compounds (4) of a library have been dispersed is illuminated with the source. The inorganic fluorescent nanoparticles (10, 11, 12) encapsulated in composite particles (1) absorb light and emit fluorescence light: the spectral and intensity of fluorescent light of the composite particle (1) allows identification of the barcode of the composite particle. If the grafted biological compound (4) is associated with a compound of interest in the sample, which is associated to a fluorescent protein (3), the protein may also absorb light and emit fluorescence light allowing the identification of the compound of interest. Preferably, the absorption wavelength of the fluorescent protein (3) is selected outside the absorption wavelength of inorganic fluorescent nanoparticles (10, 11, 12) or the emission peak of the fluorescent protein (3) is selected outside the spectral ranges of the inorganic fluorescent nanoparticles (10, 11, 12): in both situations, selecting excitation light or fluorescent light allows to determine if light is emitted by the fluorescent protein (3) or by inorganic fluorescent nanoparticles (10, 11, 12).

In this disclosure, the reader is used in combination with an assay kit and may be included in any device allowing to separate at an individual level the composite particles (1) dispersed in the sample, thus allowing to identify individually each couple of fluorescent light emitted by the fluorescent protein (3) and fluorescent light emitted by the composite particle (1) in immediate proximity of the fluorescent protein (3).

In an embodiment, two illumination sources are used: a first illumination source for excitation of fluorescence of inorganic fluorescent nanoparticles (10, 11, 12) - typically a UV light source - and a second illumination source for excitation of fluorescent protein (3) - typically a green light source. In a preferred embodiment, the first illumination source is not absorbed by the fluorescent protein (3) and the second illumination source is not absorbed by the inorganic fluorescent nanoparticles (10, 11, 12).

It has to be noted that using quantum dots as inorganic fluorescent nanoparticles (10, 11, 12) allows to use a single illumination source for all quantum dots. Indeed, quantum dots absorb all wavelength having an energy larger than their bandgap - they are high pass filters. In particular, a single UV light source may be used for all inorganic fluorescent nanoparticles (10, 11, 12) leading to simultaneous emission of fluorescence lights in all emission spectra. Such a result cannot be achieved with organic fluorophore which require to be excited at a precise wavelength: when a mixture of organic fluorophores is illuminated with a single UV light source, only some fluorophores will be excited... and likely emit light of comparable wavelength, which cannot be separated easily.

A first type of configuration for testing is to include the reader in a traditional cytometry flow system. The sample under assay is introduced in a cytometry flow so that composite particles (1) of the library will pass one by one inside the reader to be illuminated and eventually detected as positive if the fluorescent protein (3) emits light. Then, the fluorescent light emitted by the composite particle (1) - spectrum and intensity - allows identification of the compound of interest.

In a second type of configuration for testing, the sample is spread over a surface and the whole surface is placed in the reader. Here, spreading allows to separate spatially composite particles (1) in the sample, so that they can be imaged individually, as if each one was in a pixel. Under illumination, a pixel emitting light by the fluorescent protein (3) will be detected and identified by the fluorescent light - spectrum and intensity - of the composite particle (1) located in the same pixel. In this type of configuration, illumination light may be conducted by a light guide from the illumination source to the surface of analysis.

## BRIEF DESCRIPTION OF THE DRAWINGS

Figure 1 is a diagram representing a complex comprising a composite particle (1) comprising several types of inorganic fluorescent nanoparticles (10, 11, 12) encapsulated in an inorganic matrix. The composite particle (1) is surface functionalized with a particular allergen as an example of a biological compound (4). An immunoglobulin (2) is bound to the allergen and a fluorescent protein (3) is associated to the immunoglobulin (2).

Figure 2 shows the relative fluorescence intensity I (logarithmic unit) as a function of the detection channel (connected to the wavelength) in an experiment comparing, on the one hand, surface-functionalized composite particles (1) with a mixture of peanut proteins (black squares on a continuous line), and on the other hand non-surface-functionalized composite particles (1) (black circles on a dotted line), brought into contact with a biological sample. This experience demonstrates the detection efficiency. A sensitivity of 1 ng.mL$^{-1}$ was reached.

Figure 3 shows the fluorescence intensity (normalized) as a function of the wavelength (in nanometer) of 12 types of inorganic fluorescent nanoparticles (10, 11, 12), the fluorescent peaks of which are measured at 513 nm, 526 nm, 550 nm, 560 nm, 580 nm, 607 nm, 622 nm, 633 nm, 650 nm, 668 nm, 688 nm and 702 nm. The widths of the emission peaks are very narrow, thereby easily distinguishing the light emitted by fluorescence from the different types of inorganic fluorescent nanoparticles (10, 11, 12). These inorganic fluorescent nanoparticles (10, 11, 12) have a nanoplatelet structure. They are encapsulated in an inorganic matrix - typically a metal oxide such as silica, alumina, titanium oxide or a mixture - in order to protect them from oxidation and/or degradation induced by high irradiation powers, resulting in composite particles (1).

Figure 4 is a scanning electron microscopy image of a population of composite particles (1) ((4a) with magnification on some composite particles (1) for viewing the inorganic fluorescent nanoparticles (10, 11, 12) in the form of platelets, scale bar of 1 μm - (4b) in transmission, scale bar of 200 nm). It can be seen in this figure that the inorganic fluorescent nanoparticles (10, 11, 12) are distributed throughout the volume of the composite particle.

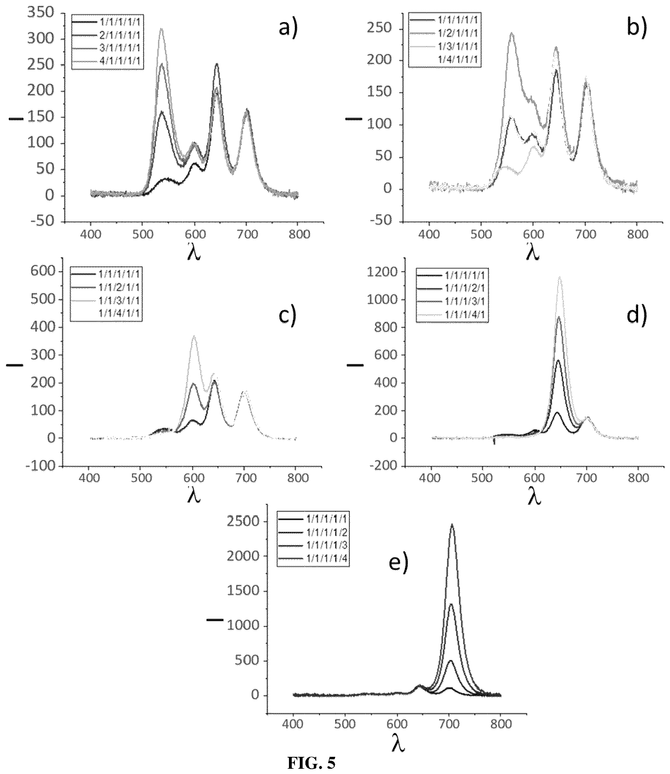

Figure 5 shows the fluorescence spectra (intensity I scaled to unity for the wavelength of 700 nm) as functions of the wavelength (in nanometer). It clearly appears that the fluorescence intensities are in proportion to the concentrations of inorganic fluorescent nanoparticles (10, 11, 12), and are well separated spectrally and in intensity.

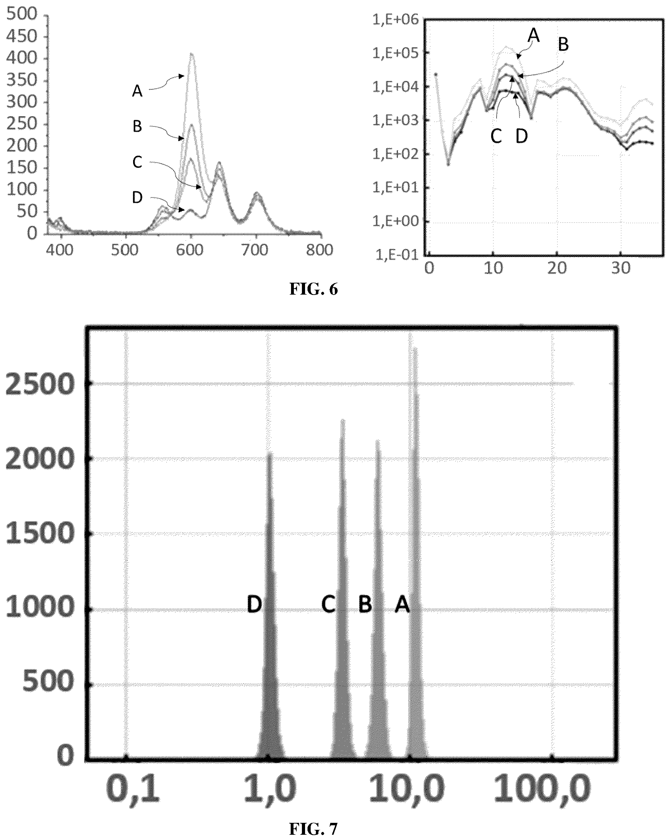

Figure 6 (on the left) exhibits the fluorescence spectrum (intensity I scaled to unity for the wavelength of 700 nm) as a function of wavelength (in nanometer) for the four populations of example 3. The right figure represents the average values of fluorescence intensity measured by flow cytometry when the particles of the four populations are employed (320 nm excitation laser, Sony ID7000 cytometer), as a function of the spectrometer acquisition channel (a wavelength measurement). It is therefore possible to measure the ratios between the channels associated with the wavelengths of the different types of inorganic fluorescent nanoparticles (10, 11, 12). For example, the ratio of channel 20 (deep red at 702 nm) and channel 8 (yellow at 560 nm) is constant for the four populations.

Figure 7 shows the ratio of the channel 12 (orange to 607 nm) and the channel 20 (deep red to 702 nm) in the form of a histogram, for the four populations of example 3.

## EXAMPLES

The present invention is further illustrated by the following examples.

Example 1: Suitable inorganic fluorescent nanoparticles (10, 11, 12)

A series of 12 fluorescent nanoparticles have been prepared, with the following characteristics.

Table I: 12 types of fluorescent particles.

| | Composition | Structure | Emission peak (nm) | FWHM (nm) |
|---|---|---|---|---|
| 1 | CdSeS/CdS/ZnS | Nanoplatelet | 513 | 28 |
| 2 | CdSeS/CdS/ZnS/CdZnS | Nanoplatelet | 526 | 27 |
| 3 | CdSe/ZnS | Nanoplatelet | 550 | 24 |
| 4 | CdSeS/ZnS/CdS/ZnS | Nanoplatelet | 560 | 25 |
| 5 | CdSe/ZnS | Nanoplatelet | 580 | 22 |
| 6 | CdSe/ZnS | Nanoplatelet | 607 | 23 |
| 7 | CdSeS/CdZnS | Nanoplatelet | 622 | 24 |
| 8 | CdSe/CdZnS | Nanoplatelet | 633 | 19 |
| 9 | CdSe/CdZnS | Nanoplatelet | 650 | 19 |
| 10 | CdSe/CdZnS/ZnS | Nanoplatelet | 668 | 29 |
| 11 | CdSe/CdS/ZnS | Nanoplatelet | 688 | 25 |
| 12 | CdSe/CdS/ZnS | Nanoplatelet | 702 | 29 |

The fluorescence spectra of these 12 fluorescent nanoparticles are represented on figure 3 - intensity scaled to unity with the peak intensity. Within these 12 fluorescent nanoparticles, one can easily select 4, 5 or 6 items with the following features: emission peak of an item is outside the FWHM of neighboring items and fluorescence peak of a fluorescent protein (3) is outside the FWHMs of all items, thus obtaining a set of fluorescent particles to prepare barcoded particles.

Example 2: library of 1024 populations - sub-library of 16 populations

In the set of fluorescent particles prepared in example 1, particles with emission peaks at 526 nm (#2), 560 nm (#4), 607 nm (#6), 650 nm (#9) and 702 nm (#12) are selected: N=5. For each type of particles, a scale of weight fraction is defined with four levels according to the following series: 1, 2.03, 3.42, 5.17: P=4.

Then composite particles (1) are prepared with the following procedure. 10 ml of dispersion $A_{ij}$ in a basic aqueous solution is prepared with a weight fraction of particle #i of $C_{ij}$, for all ij combinations, *i.e.,* 1024 solutions. For instance, a composite particle (1) comprising 1% of particle #2 - level 1 -, 2.03% of particle #4- level 2 -, 2.03% of particle #3 - level 2 -, 1% of particle #4 - level 1 - and 5.17% of particle #5 - level 4 - is noted (1,2,2,1,4). In this example, the null weight fraction is not used.

The dispersion $A_{ij}$ is then used in the process disclosed in international patent application PCT/EP2018/064439 to yield composite particles (1) comprising the fluorescent particles in the same repartition as in the solution. For all 1024 samples, the volume of solution $A_{ij}$ comprising fluorescent particles and the properties - concentration of inorganic precursors, volume - of the solution B comprising matrix precursors are kept similar, so that weight fractions in all samples are defined according to the same scale of weight fractions. In other words, a weight fraction of 1% of particle #1 in a solution $A_{ij}$ leads to the same weight fraction amount in the composite particles (1).

The concentration of inorganic precursors, the volumes of solutions $A_{ij}$ and B, and the process conditions are designed so that the weight fraction in composite particles (1) is the same as the weight fraction in the solution $A_{ij}$.

Last, each population is surface modified with a biological compound (4), in this example allergens. Finally, a library is obtained, in which each population comprises a specific barcode associated to an allergen.

Figure 2 shows the relative fluorescence intensity I (logarithmic unit) as a function of the detection channel (connected to the wavelength) in an experiment comparing, on the one hand, surface-functionalized composite particles (1) with a mixture of peanut proteins (black squares on a continuous line), and on the other hand non-surface-functionalized composite particles (1) (black circles on a dotted line), brought into contact with a biological sample comprising IgE (f13) - Peanut binding with peanut proteins - and a Green Fluorescent Protein (GFP). This experience demonstrates the detection efficiency. A sensitivity of 1 ng.mL$^{-1}$ was reached.

The allergens used in this example are the following:

Table II: a sub-library of 16 populations corresponding to 16 allergens.

| Population | Allergen - target IgG |
|---|---|
| (1, 1, 1, 1, 1) | Peanut - IgE (f13) |
| (2, 1, 1, 1, 1) | Oatmeal - IgG (f7) |
| (3, 1, 1, 1, 1) | Cat dander - IgG (e1) |
| (4, 1, 1, 1, 1) | Horse dander - IgG (e3) |
| (1, 2, 1, 1, 1) | Dog dander - IgG (e5) |
| (1, 3, 1, 1, 1) | Chicken feathers - IgG (e85) |
| (1, 4, 1, 1, 1) | Duck feathers - IgG (e86) |
| (1, 1, 2, 1, 1) | Canary bird feathers - IgG (e201) |
| (1, 1, 3, 1, 1) | Parrot feathers - IgG (e213) |
| (1, 1, 4, 1, 1) | Cultivated oat - IgG (g14) |
| (1, 1, 1, 2, 1) | Cultivated wheat - IgG (g15) |
| (1, 1, 1, 3, 1) | Egg white - IgG (f1) |
| (1, 1, 1, 4, 1) | Soybean - IgG (f14) |
| (1, 1, 1, 1, 2) | milk (alpha-lactalbumin) - IgG (f76) |
| (1, 1, 1, 1, 3) | milk (beta-lactoglobulin) - IgG (f77) |
| (1, 1, 1, 1, 4) | milk (casein) - IgG (f78) |

For population (3, 1, 1, 1, 1), the concentration of inorganic fluorescent nanoparticles (10, 11, 12) emitting at 526 nm is 3.42 times that of population (1, 1, 1, 1, 1). Figure 5 shows the fluorescence spectra (normalized to unity for the wavelength of 700 nm) as functions of the wavelength $\lambda$ (in nanometer). It clearly appears that the fluorescence intensities are in proportion to the concentrations of inorganic fluorescent nanoparticles (10, 11, 12).

By measuring the fluorescence spectrum of an isolated composite particle (1) in a flow cytometry analysis, a unique spectrum associated with the biological compound (4) present on the surface of the composite particle (1) is identified.

Example 3: library of 1024 populations - sub-library of 4 populations

Another sub-library of 4 populations (A to D) was prepared. Each population contains the same concentration - scaled to 1 - of inorganic fluorescent nanoparticles (10, 11, 12) emitting at 405 nm (core/shell nanospheres of ZnSe/ZnS, with a core of 2,7 nm diameter and a shell thickness of 2 nm), 560 nm (#4), 650 nm (#9) and 702 nm (#12). On the other hand, the concentration of inorganic fluorescent nanoparticles (10, 11, 12) emitting at 607 nm (#6) is different for each population: 1 (D), 2 (C), 4 (B) and 8 (A). For the population (A), the weight fraction of the inorganic fluorescent nanoparticles (10, 11, 12) in the composite particles (1) is about 15%. Here, N=5 and P=4 again.

Figure 6 (on the left) exhibits the fluorescence spectrum (scaled to 1 for 700 nm) as a function of wavelength (in nanometer) for the four populations of example 3. The right figure represents the average values of fluorescence intensity measured by flow cytometry when the particles of the four populations are employed (320 nm excitation laser, Sony ID7000 cytometer), as a function of the spectrometer acquisition channel (a wavelength measurement). It is therefore possible to measure the ratios between the channels associated with the wavelengths of the different types of inorganic fluorescent nanoparticles (10, 11, 12). For example, the ratio of channel 20 (deep red at 702 nm) and channel 8 (yellow at 560 nm) is constant for the four populations.

Figure 7 shows the ratio of the channel 12 (orange to 607 nm) and the channel 20 (deep red to 702 nm) in the form of a histogram, for the four populations of example 3.

Thus, it is possible to clearly distinguish the composite particle populations prepared on the basis of the concentrations of inorganic fluorescent nanoparticles (10, 11, 12) contained therein.

**REFERENCES NUMERIQUES**

1 - Composite particle
2 - Immunoglobulin
3 - Fluorescent protein
4 - Biological compound (biomolecule, for example allergen, nucleic acid or protein, including recombinant protein)
10, 11, 12 - inorganic fluorescent nanoparticles

**Claims**

1. A library of barcoded particle populations in which:

   • the barcoded particles are composite particles (1) comprising at least one type of inorganic fluorescent nanoparticle selected from a list of N types of inorganic fluorescent nanoparticles (10, 11, 12), these inorganic fluorescent nanoparticles (10, 11, 12) being encapsulated in an inorganic material and N being an integer greater than or equal to 3;
   • each population is defined by an N-tuple, wherein the element i of the N-tuple is the weight fraction of the type i of inorganic fluorescent nanoparticle in the composite particles (1), defining a fluorescence spectrum $I_{(C1,\ldots,Ci,\ldots CN)}(\lambda)$ for that population;
   • each type of the N types of inorganic fluorescent nanoparticles (10, 11, 12) is encapsulated in at least one population; and
   • at least one type of inorganic fluorescent nanoparticle is encapsulated in at least two populations with different weight fractions.

2. The library according to claim **1,** wherein the inorganic fluorescent nanoparticles (10, 11, 12) are distributed throughout the volume of each composite particle (1).

3. The library according to claim **1** or claim **2,** wherein the weight fraction of the inorganic fluorescent nanoparticles (10, 11, 12) in the composite particles (1) of at least one population is greater than or equal to 10%.

4. The library according to any one of claims **1** to **3,** wherein the weight fraction of the type i of the inorganic fluorescent nanoparticles (10, 11, 12) in the composite particles (1) is selected from a P-tuple $(C_{i1},...C_{iP})$ of predetermined concentrations, wherein P is greater than or equal to 3.

5. The library according to any one of claims **1** to **4,** wherein the inorganic fluorescent nanoparticles (10, 11, 12) are semi-conductive nanoparticles.

6. The library according to any one of claims **1** to **5,** wherein the composite particles (1) are surface-modified with biological compounds (4).

7. An assay kit comprising a library of barcoded particle populations according to claim **6** and at least one fluorescent protein (3).

8. The assay kit according to claim **7,** wherein the composite particles (1) are surface modified with allergens and the at least one fluorescent protein (3) is able to bind with immunoglobulins (2), preferably the at least one fluorescent protein (3) is selected from the group of Fluorescein Isothiocyanate (FITC), Peridinin-Chlorophyll Protein Complex (PerCP), Rhodamine, Green Fluorescent Protein (GFP), Yellow Fluorescent Protein (YFP), Propium Iodide, Phycorythrin, Sulforhodamine, Cy5, Cy7, Cy5.5, Cy7.5, DRAQ7.

9. The assay kit according to claim **7,** wherein the composite particles (1) are surface modified with a biomolecule (4) able to bind with an immunoglobulin (2) produced after exposure to a virus and the at least one fluorescent protein (3) is able to bind with immunoglobulins (2).

10. The assay kit according to claim **7,** wherein the composite particles (1) are surface modified with a biomolecule (4) able to bind with an envelope glycoprotein of a virus and the at least one fluorescent protein (3) is able to bind with said virus.

11. Device for biological assay configured to separate at an individual level the composite particles (1) from an assay kit according to any one of claims **7** to **10,** dispersed in a biological sample and comprising a reader comprising:

  • at least one illumination source; and
  • a light detector measuring light intensity and coupled to a spectrometer,

  wherein the at least one illumination source triggers fluorescence of the inorganic fluorescent nanoparticles (10, 11, 12) encapsulated in composite particles (1) and fluorescence of the at least one fluorescent protein (3).

12. Device for biological assay according to claim **11,** wherein separation at an individual level of the composite particles (1) is operated by introduction of the biological sample in a flow cytometer.

13. Device for biological assay according to claim **11,** wherein separation at an individual level of the composite particles (1) is operated by spreading of the biological sample on a surface.

FIG. 1

FIG. 2

**FIG. 3**

**FIG. 4**

**FIG. 5**

EP 4 343 329 A1

**FIG. 6**

**FIG. 7**

**Europäisches Patentamt**
**European Patent Office**
**Office européen des brevets**

# EUROPEAN SEARCH REPORT

Application Number

EP 23 15 9082

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Mingyong Han ET AL: "Quantum-dot-tagged microbeads for multiplexed optical coding of biomolecules", Nature Biotechnology volume 19, pages631-635, 1 January 2001 (2001-01-01), XP93073659, Retrieved from the Internet: URL:https://www.nature.com/articles/nbt0701_631 [retrieved on 2023-08-15] | 1-13 | INV. G01N33/58 |
| Y | * the whole document * * figure 1 * | 1-13 | |
| X | XU YUESHUANG ET AL: "Emerging barcode particles for multiplex bioassays", SCIENCE CHINA MATERIALS, vol. 62, no. 3, 10 September 2018 (2018-09-10), pages 289-324, XP093001405, ISSN: 2095-8226, DOI: 10.1007/s40843-018-9330-5 Retrieved from the Internet: URL:http://link.springer.com/content/pdf/10.1007/s40843-018-9330-5.pdf> | 1-13 | |
| Y | * the whole document * * page 290, section "Quantum dot encoding" * | 1-13 | TECHNICAL FIELDS SEARCHED (IPC) G01N |
| X | WO 2018/220160 A1 (NEXDOT [FR]) 6 December 2018 (2018-12-06) | 11-13 | |
| Y | * the whole document * * claims 1-21 * | 1-13 | |

-/--

The present search report has been drawn up for all claims

| Place of search | Date of completion of the search | Examiner |
|---|---|---|
| The Hague | 16 August 2023 | Jenkins, Gareth |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 23 15 9082

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SHANG-HSIU HU ET AL: "Stable Encapsulation of Quantum Dot Barcodes with Silica Shells", ADVANCED FUNCTIONAL MATERIALS, vol. 20, no. 21, 9 November 2010 (2010-11-09), pages 3721-3726, XP055292858, DE ISSN: 1616-301X, DOI: 10.1002/adfm.201000711 | 11-13 | |
| Y | * the whole document * <br> * figure 1 * <br> ----- | 1-13 | |

TECHNICAL FIELDS
SEARCHED       (IPC)

The present search report has been drawn up for all claims

| Place of search | Date of completion of the search | Examiner |
|---|---|---|
| The Hague | 16 August 2023 | Jenkins, Gareth |

EPO FORM 1503 03.82 (P04C01)

## ANNEX TO THE EUROPEAN SEARCH REPORT
## ON EUROPEAN PATENT APPLICATION NO.

EP 23 15 9082

This annex lists the patent family members relating to the patent documents cited in the above-mentioned European search report.
The members are as contained in the European Patent Office EDP file on
The European Patent Office is in no way liable for these particulars which are merely given for the purpose of information.

16-08-2023

| Patent document cited in search report | | Publication date | Patent family member(s) | Publication date |
|---|---|---|---|---|
| WO 2018220160 A1 | | 06-12-2018 | CN 111201301 A | 26-05-2020 |
| | | | DK 3630917 T3 | 10-10-2022 |
| | | | EP 3630917 A1 | 08-04-2020 |
| | | | EP 4101907 A1 | 14-12-2022 |
| | | | ES 2929537 T3 | 30-11-2022 |
| | | | JP 2020523432 A | 06-08-2020 |
| | | | KR 20200023295 A | 04-03-2020 |
| | | | PL 3630917 T3 | 14-11-2022 |
| | | | TW 201905161 A | 01-02-2019 |
| | | | TW 201905162 A | 01-02-2019 |
| | | | WO 2018220160 A1 | 06-12-2018 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2018064439 A